# EUROPEAN PATENT APPLICATION

(11) **EP 1 978 459 A1**
(43) Date of publication of application: **08.10.2008**
(21) Application number: 07105664.2
(22) Date of filing: 04.04.2007
(51) Int. Cl.: G06F 19/00

(54) **Determination of patient specific biomarkers**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The invention relates to an approach for the identification of biomarkers that reflect patient-to-patient variations with respect to a given disease. According to this method, the biomarkers are found by the identification of model parameters on which selected state variables of the model depend with high sensitivity in a multi-parametric mathematical model of the disease.

## Description

The invention relates to a method for the determination of biomarkers that reflect patient-to-patient variations with respect to a specific disease.

For many diseases there is a rapidly increasing knowledge about the mechanisms behind them that can be used for better predictions about their course. The WO 2005 007744 A1 describes in this respect a simulation environment for complex biological systems and diseases. While it is important to understand the characteristic and unique features of a specific disease, an optimal therapy requires as well a sufficient consideration of patient-to-patient variations as they appear in each biological system.

Based on this background it was an object of the present invention to provide means that allow an improved understanding of diseases and a better planning of therapies.

This object is achieved by a method according to claim 1, by a computer program according to claim 5, and by a record carrier according to claim 6. Preferred embodiments are disclosed in the dependent claims.

The method according to the present invention serves for the determination of biomarkers that reflect patient-to-patient variations with respect to some given disease. The method comprises the following steps:
- The identification of model parameters in a (multi-)parametric mathematical model of the disease such that selected state variables of the model depend with highest sensitivity on these parameters. The selected state variables of the model may for example be concentrations of particular molecules in blood or tissue, physiological parameters like blood pressure or the like. The "sensitivity" of a quantity that continuously depends on some parameter is typically defined as the (partial) derivative of this quantity with respect to said parameter.
- The use of the aforementioned identified parameters or the use of quantities that are derived from said parameters as the desired biomarkers.

The method has the advantage that the described biomarkers can be found by a definite, reproducible procedure that can optionally be executed automatically. Moreover, the identified parameters have a significant influence on characteristic quantities of the disease and are therefore likely to be relevant indicators of patient-to-patient variations.

The mathematical model preferably describes molecular mechanisms like chemical reactions of proteins, gene expression etc. Such models become more and more available due to the increasing biochemical knowledge.

Moreover, the mathematical model is preferably formulated in terms of ordinary differential equations (ODE) and/or of Bayesian networks. Reference is made in this context to the relevant literature for more information on these mathematical tools, e.g. to an article of M. Bentele et al. ("Mathematical modeling reveals the threshold mechanism in CD95-induced apoptosis", The Journal of Cell Biology (v 166 (6) pp 839-851)) that describes the application of ODE-based modeling for the understanding of large signal transduction networks and attempts to identify the threshold mechanism in the regulation of apoptosis. Moreover, some applications of Bayesian networks for the description of biological models have also been described in literature (e.g. Friedman, N., "Inferring cellular networks using probabilistic graphical models", Science 303(5659):799-805 (2004); Smith, A., Jarvis, E. and Hartemink, A., "Evaluating functional network inference using simulations of complex biological systems", Bioinformatics, Volume 18, Suppl. 1, pp S216-S224 (2002); Hartemink, A., "Bayesian Networks and Informative Priors: Transcriptional Regulatory Network Models", In Bayesian Inference for Gene Expression and Proteomics, Do, K.-A., Müller, P., & Vannucci, M., eds. Cambridge University Press: Cambridge, UK. pp. 401-424 (2006)).

The identification of model parameters on which selected state variables of the model depend with highest sensitivity is optionally done by sensitivity analysis as it is known from system theory. Reference is made in this context to the relevant literature for more information on this technology (e.g. to Saltelli, A., M. Ratto, S. Tarantola and F. Campolongo, "Sensitivity Analysis for Chemical Models", Chemical Reviews, 105(7) pp 2811 - 2828 (2005); Cacuci, Dan G., Mihaela Ionescu-Bujor, Michael Navon, "Sensitivity And Uncertainty Analysis: Applications to Large-Scale Systems" (Volume II), Chapman & Hall (2005).

The invention further relates to a computer program for enabling carrying out a method of the kind described above.

Finally, the invention comprises a record carrier, for example a floppy disk, a hard disk, or a compact disc (CD), on which a computer program of the aforementioned kind is stored.

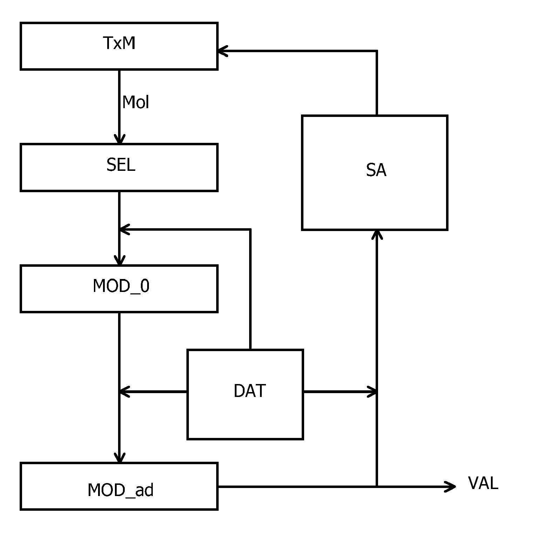

These and other aspects of the invention will be apparent from and elucidated with reference to the embodiment(s) described hereinafter. These embodiments will be described by way of example with the help of the accompanying single drawing which shows a float chart illustrating the application of sensitivity analysis in the design of mathematical disease models.

Systems biology applies quantitative, mechanistic modeling to study genetic networks, signal transduction pathways and metabolic networks. Mathematical models of biochemical networks can look very different. An important reason is that the purpose and application of a model are essential for the selection of the best mathematical framework. Fundamental aspects of selecting an appropriate modeling framework and a strategy for model building can be found in literature (e.g. van Riel NA, Dynamic modelling and analysis of biochemical networks: mechanism-based models and model-based experiments; Brief Bioinform. 2006 Nov 14).

There are several factors that are influencing the outcomes of the predictions in current modeling methods including variability in patients, variability between patient groups and technical variability due to indirect measurement techniques. Present methods for biomarker discovery require large data sets including tissue and blood expression profiles, or proteomics studies in blood samples. Such methods rely greatly on large data sets that require substantial investment and are logistically difficult. Additionally biological variability of changes in molecular profiles of the patients makes it difficult to identify relevant patterns (biomarker sets) using small patient groups. The present invention deals with biomarker discovery and identification of specific markers that reflect major variability between patients. It allows to overcome the need of costly investments in high-throughput methods and is aimed to identify the important molecular entities from the existing knowledge of the molecular entities and their properties. The identified potential molecular entities are validated using in vitro and in vivo experiments.

The invention proposes to use the existing knowledge of the interactions and effects that different molecular entities are playing in a disease as a base for the model. Such interactions are thereafter described using mathematical means in the form of ODE (ordinary differential equations) or Bayesian networks. Quantitative parameters describing the effects of different components on the others are used to perform a sensitivity analysis of the whole system to identify potential clinically relevant biomarkers. These markers can be later on used in clinical diagnosis and therapy planning and are the key indicators of the disease progression.

The Figure shows a general flow chart of the model development and of the determination of biomarkers according to the present invention. The proposed approach requires a disease model described in terms of molecules and their effects on each other. Such model is developed using the information on the interactions of biomolecules and experimental values describing those interactions and not the statistical patterns. An initial model or network (MOD_0) of a disease is for example created using existing knowledge from publications in the public domain, e.g. via Text Mining (TxM) and initial preselection (SEL) with respect to certain biomolecules (MOL). Data (DAT) from experiments can also be used, particularly to fill the initial model MOD_0 thereafter with quantitative parameters describing the interactions between the components of the network, yielding an adapted model or network (MOD_ad).

The multi-parametric sensitivity analysis (SA) of the obtained model MOD_ad is then performed to identify most sensitive nodes in such system. Those nodes are identified as biomarkers. For each particular patient such set of biomarkers is used to identify patient specific differences and provides the guidelines for identifying relevant measurements through CDSS (clinical decision support systems) indicating the following measurements that need to be performed.

In summary, the invention describes a method aimed to identify the biomarkers in different disease areas that reflect patient-to-patient variations. The invention is aimed to help to identify biomarkers that are patient sensitive and that can be used for diagnostic purposes for monitoring and therapy planning. The invention uses the sensitivity analysis method from the control systems theory.

Finally it is pointed out that in the present application the term "comprising" does not exclude other elements or steps, that "a" or "an" does not exclude a plurality, and that a single processor or other unit may fulfill the functions of several means. The invention resides in each and every novel characteristic feature and each and every combination of characteristic features. Moreover, reference signs in the claims shall not be construed as limiting their scope.

## Claims

1. A method for the determination of biomarkers that reflect patient-specific variations with respect to a disease, comprising the following steps:
- identification of parameters of a parametric mathematical model of the disease on which given state variables of the model depend with highest sensitivity;
- using the identified parameters or quantities derived therefrom as biomarkers.

2. The method according to claim 1,
**characterized in that** the mathematical model describes molecular mechanisms.

3. The method according to claim 1,
**characterized in that** the mathematical model is formulated in terms of ordinary differential equations and/or Bayesian networks.

4. The method according to claim 1,
**characterized in that** the identification is done by sensitivity analysis.

5. A computer program for enabling carrying out a method according to claim 1.

6. A record carrier on which a computer program according to claim 5 is stored.
